# EUROPEAN PATENT SPECIFICATION

(11) **EP 4 134 325 B1**
(45) Date of publication and mention of the grant of the patent: **14.10.2026**
(21) Application number: 21783863.0
(22) Date of filing: 02.04.2021
(51) Int. Cl.: B65D 51/18, B65D 39/00, B65D 41/04, B65D 1/02

(54) **FRAGRANCE LIQUID CONTAINER**
BEHÄLTER FÜR FLÜSSIGE DUFTSTOFFE
RÉCIPIENT DE PARFUM LIQUIDE

(30) Priority: 08.04.2020 CN 202020501917 U; 08.04.2020 CN 202020501910 U; 30.06.2020 CN 202021241991 U; 06.11.2020 CN 202022551446 U
(43) Date of publication of application: 15.02.2023
(73) Proprietor: Dalian Talent Gift Co., Ltd., Dalian, Liaoning 116200 (CN)
(72) Inventor: WANG, Lixin, Dalian, Liaoning 116011 (CN)
(74) Representative: Patentanwälte Ruff, Wilhelm, Beier, Dauster & Partner mbB
(86) International application number: PCT/CN2021/085287
(87) International publication number: WO 2021/204081

(56) References cited:
- CN-U- 201 580 664
- CN-U- 202 590 010
- CN-U- 203 154 387
- CN-U- 211 919 540
- JP-A- 2019 123 554
- KR-A- 20160 149 367
- KR-A- 20160 149 367
- US-A- 4 928 881

## Description

### FIELD OF THE INVENTION

The invention relates to a reed diffuser container, and more particularly, the reed diffuser container according to the invention can prevent accidental opening by children and leakage of the scented liquid while allowing the scented liquid to volatilize.

### BACKGROUND

Reed diffuser is commonly used by people in the office and at home. It has the effects of refreshing the air and enhancing the physical and psychological well-being. The scented liquid is usually contained in an open container, in contact with a carrier (such as reed, grass flower, cotton string, etc.) inserted through the opening of the bottle, and transported to the end of the carrier located outside the bottle by means of the wicking action of the carrier, and finally diffuses into the atmosphere through evaporation or volatilization, scenting the air.

Most of the reed diffuser containers on the market are simple in structure, and after the carrier (such as reeds) is inserted to initiate the use of the product, the opening is kept open and would not be closed again. In this case, although the opening facilitates the insertion and replacement of the carrier such as the reed, once the container was accidentally turned over, the scented liquid would flow out, causing waste and pollution, and the excessively strong odor would be irritating to the human body. In addition, in some cases, the opening of the reed diffuser container is closed by a cover, but the cover can be easily opened by children or easily fall off under the action of external forces such as bumps, etc., which would result in leakage of the scented liquid and thus accidental eating and misuse by children and other safety hazards. And this will also cause waste and pollution of the scented liquid. JP2019123554A discloses a reed diffuser container

### SUMMARY

The invention aims to provide a reed diffuser container, which can not only allow the scented liquid to volatilize and diffuse the fragrance, but also prevent children from accidentally opening it and prevent the liquid from flowing out when the reed diffuser container is accidentally overturned. The invention is directed to a reed diffuser container according to claim 1.

According to a first embodiment of the present disclosure a reed diffuser container is provided. The reed diffuser container comprises a bottle body for containing the scented liquid, a safety inner cover and an outer cover. The bottle body is provided with a bottle mouth. The safety inner cover is provided with a stick insertion portion, which can include one or more insertion holes to allow the stick to pass through the safety inner cover and enter the bottle body. The insertion holes can abut closely with the sticks. The safety inner cover has an upper cover portion and a circumferentially extending skirt. The inner surface of the skirt is provided with a protrusion that can be snap-fitted with the engaging groove provided on the bottle body below the bottle mouth, such that the safety inner cover can close the bottle mouth in a liquid-tight manner. In an assembled state, the outer cover is fixed to the bottle body and covers the safety inner cover entirely from the outside.

According to a second embodiment of the present disclosure a reed diffuser container is provided. The reed diffuser container comprises a bottle body for containing the scented liquid, a safety inner cover and an outer cover assembly. The safety inner cover is provided with a stick insertion portion, which can include one or more insertion holes to allow the stick to pass through the safety inner cover and enter the bottle body. The insertion holes can abut closely with the sticks. The safety inner cover tightly closes the bottle mouth provided on the bottle body in a liquid-tight manner. The outer cover assembly can be detachably connected to the bottle mouth from the outside of the safety inner cover. The outer cover assembly comprises a first cover member and a second cover member. In use, the first cover member is sleeved outside of the second cover member. Both the first cover member and the second cover member have an upper cover portion and a circumferentially extending skirt, and the upper cover portions of the first cover member and the second cover member are provided with aligned central through-holes to expose the stick insertion portion on the safety inner cover. The first cover member and the second cover member are provided with matching engaging features through which the first cover member and the second cover member can engage with each other. In an engaged state, the second cover member can rotate along with the rotation of the first cover member; in a disengaged state, the first cover member is configured to be rotatable relative to the second cover and movable in the longitudinal direction.

According to a third embodiment of the present disclosure a reed diffuser container is provided. The reed diffuser container comprises a bottle body for containing the scented liquid, a safety inner cover and an outer cover. The bottle body is provided with a bottle mouth, and the safety inner cover is provided with a reed insertion portion, which can include one or more insertion holes to allow the stick to pass through the safety inner cover and enter the bottle body. The insertion holes can abut closely with the sticks. The safety inner cover comprises an upper cover portion and a plunger portion, wherein the plunger portion can extend into the bottle mouth and abut closely with the inner wall of the bottle mouth in a liquid-tight manner, and the upper cover portion can fit closely on the flange provided on the bottle mouth. The outer cover is connected to the bottle body from the outside of the safety inner cover, and comprises an upper cover portion and a circumferentially extending skirt. The upper cover portion of the outer cover is provided with a central through-hole. The inner surface of the skirt of the outer cover is provided with a protrusion, which engages beneath the flange to fix the outer cover to the bottle mouth. The outer cover presses the safety inner cover against the bottle body, accordingly.

According to a fourth embodiment of the present disclosure a reed diffuser container is provided. The reed diffuser container comprises a bottle body for containing the scented liquid, a sealing cover, an inner cover and an outer cover. The bottle body is provided with a bottle mouth, and the sealing cover is provided with a stick insertion portion, which can include one or more insertion holes to allow the stick to pass through the sealing cover. The insertion holes can abut closely with the sticks. Both the outer cover and the inner cover include an upper cover portion and a circumferentially extending skirt. The upper cover portions of the outer cover and the inner cover are provided with aligned central through-holes. The inner cover can be engaged with the bottle body and is sleeved inside the outer cover. In a disengaged state of the outer cover and the inner cover, the outer cover is rotatable relative to the inner cover and movable in the longitudinal direction. In an engaged state, the outer cover can rotate together with the inner cover. The sealing cover has a disc-shaped body which covers the central through-hole of the inner cover. The sealing portion of the sealing cover extends radially outward from the lower surface of the body, and forms an engaging groove with the lower surface of the body. The engaging groove can snap-fit with the edge of the central through-hole of the inner cover.

The reed diffuser container according to the present invention provides effective sealing of the bottle mouth through the configuration of cover components. Due to the safety inner cover or sealing cover, the scented liquid would not leak out at any time even if the container was accidentally overturned. In addition, by providing a cover structure that is not easy for children to open, it can prevent children from accidentally opening the bottle cover and causing danger.

### BRIEF DESCRIPTION OF THE DRAWINGS

The invention and its advantageous embodiments will be explained in more detail below by way of example in conjunction with schematic drawings. In the drawings:
Fig. 1 is an exploded schematic view of a reed diffuser container according to a first embodiment of the present disclosure
Fig. 2 is a schematic view of the reed diffuser container according to the first embodiment of the present disclosure in an assembled state;
Fig. 3 is a plan view of an example of an insertion hole on a safety inner cover according to the first embodiment of the present disclosure
Fig. 4 is a cross-sectional view of an example of the safety inner cover according to the first embodiment of the present disclosure
Fig. 5 is an exploded schematic view of a reed diffuser container according to a second embodiment of the present disclosure
Fig. 6 is a schematic view of an assembled state of the reed diffuser container according to the second embodiment of the present disclosure
Fig. 7 is a perspective view of a first cover member of an outer cover assembly according to the second embodiment of the present disclosure
Fig. 8 is a perspective view of a second cover member of the outer cover assembly according to the second embodiment of the present disclosure
Fig. 9 is an exploded schematic view of a reed diffuser container according to a third embodiment of the present disclosure
Fig. 10 is a schematic view of an assembled state of the reed diffuser container according to the third embodiment of the present disclosure
Fig. 11 is an exploded schematic view of another example of the reed diffuser container according to the third embodiment of the present disclosure
Fig. 12 is an exploded schematic view of a reed diffuser container according to a fourth embodiment of the present disclosure
Fig. 13 is a perspective view of an intermediate sealing cover according to the fourth embodiment of the present disclosure
Fig. 14 is a cross-sectional view of the intermediate sealing cover according to the fourth embodiment of the present disclosure
Fig. 15 is a perspective view of an outer cover according to the fourth embodiment of the present disclosure

### DETAILED DESCRIPTION OF EMBODIMENTS

It should be noted that the features in the drawings are not necessarily drawn to scale. In fact, the dimensions of the features can be increased or decreased as desired. It should be noted that the various embodiments of the present invention and the features of the embodiments may be combined with one another if they are not conflicting.

Orientation terms such as "upper, lower" and "top, bottom" as well as relative orientation terms such as "on", "above", "on the top surface of' are used only for ease of reference to the drawings. They do not indicate or imply that the device or element referred to must have a particular orientation or be constructed and operated in a particular orientation, or that the device or element referred to must have a fixed relative position. Orientation terms "inside, outside" refer to inside and outside the contours of the elements per se. The above terms are used for illustrative purposes only and are not intended to limit the present disclosure.

In addition, it should be noted that the use of words such as "first" and "second" to define elements is only for easily distinguishing between the elements, and is not intended to limit the present invention.

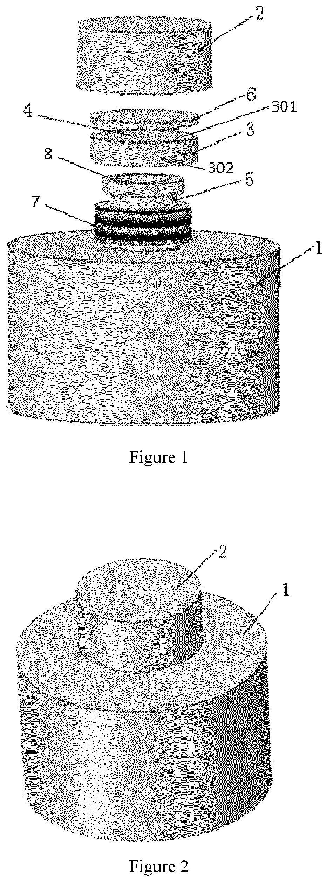

Figs. 1 and 2 illustrate a reed diffuser container according to a first embodiment of the present disclosure The reed diffuser container generally comprises a bottle body 1 for containing the scented liquid, an outer cover 2 and a safety inner cover 3. The bottle body 1 is provided with a neck, and the end of the neck is provided with a bottle mouth 8. On the neck of the bottle body 1, a recessed engaging groove 5 is provided at a distance from the bottle mouth 8. External threads 7 are arranged on the neck of the bottle body 1 below the engaging groove 5. Internal threads matching the external threads 7 are provided on the inner surface of the outer cover 2. When the diffuser bottle is not in use (i.e., the scented liquid is not volatilized), the outer cover 2 can be thread-connected with the bottle body 1, as shown in Fig. 2. At this time, the outer cover 2 entirely covers the safety inner cover 3 on the outside, keeping the appearance of the diffuser bottle concise and beautiful. When the diffuser bottle is used to volatilize the scented liquid, the outer cover 2 can be unscrewed from the bottle body 1 to expose the safety inner cover 3. Preferably, when the outer cover 2 is not thread-connected with the bottle body 1, it can be connected to the bottle body 1 by means of additional connecting members such as a tether or the like, or attached to the bottle body 1 by being inserted into a receptacle provided on the bottle body 1, so as to avoid the loss of the outer cover 2 or other problems related to storage, and facilitate the reuse of the diffuser bottle.

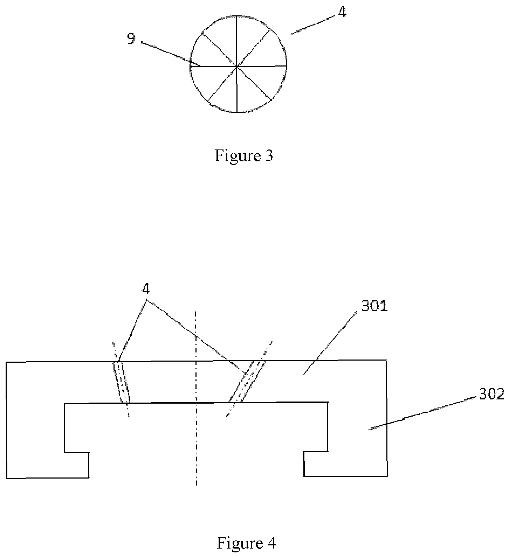

The safety inner cover 3 can be in the form of a resilient plug, which can be made of an elastomeric material such as rubber. Referring to Fig. 4, the safety inner cover 3 has an upper cover portion 301 and a circumferentially extending skirt 302. The inner surface of the skirt 302 of the safety inner cover 3 is provided with a protrusion, which can be snap-fitted with the engaging groove 5 provided on the neck of the bottle body 1, such that the safety inner cover 3 can tightly close the mouth of the bottle body 1 in a liquid-tight manner.

The upper cover portion 301 of the safety inner cover 3 is provided with a stick insertion portion through which the sticks as carriers can be inserted into the bottle body 1 and in contact with the scented liquid contained in the bottle body 1. In the illustrated embodiment, the stick insertion portion is in the form of several insertion holes 4. Before inserting the sticks, the insertion holes 4 are in a substantially closed state. When the sticks are inserted, in particular due to the elastic nature of the inner safety cover, the insertion hole 4 will expand slightly as the stick is inserted through the inner safety cover, and at least a portion of the wall of the holes is in close contact with the stick.

In an advantageous example of the present disclosure the insertion holes 4 may be configured as through-holes. Optionally, the stick insertion portion can be covered by a sealing sheet to prevent the scented liquid from leaking out through the insertion holes 4. When in use, the sealing sheet can be pierced or removed before the stick is inserted. The sealing sheet can, for example, be in the form of a film. Preferably, the sealing sheet is provided with dents or marks corresponding to the positions of the insertion holes 4 so that it can be easily pierced.

Referring to Fig. 3, in another advantageous example of the present disclosure a layer of sheet material made of a flexible material is provided in the opening of the insertion holes 4. The layer of the sheet material can cover the opening of the insertion holes 4. The sheet has several slits 9 which divide the sheet into several fan-shaped flaps. Before the stick(s) is(are) inserted, the flaps are tightly adjacent to each other to close the insertion holes together; when the stick(s) is(are) inserted, the flaps are separated from one another along the slits 9 and bent towards the bottle body, allowing the passage of the stick(s) and pressing against the stick(s).

Preferably, the diameter of the insertion holes 4 is smaller than the diameter of the stick, such that when the stick is inserted, the insertion hole 4 fits closely with the stick.

Preferably, as shown in Fig. 4, the direction in which the insertion holes 4 extend in the thickness of the upper cover portion 301 of the safety inner cover 3 is at an angle with respect to the longitudinal direction of the bottle body. The angles of the insertion holes 4 with respect to the longitudinal direction may be the same or different as required.

In an advantageous example of the present disclosure the insertion holes 4 are configured as blind holes, and will be passed through upon insertion of the stick.

In an advantageous example of the present disclosure the stick insertion portion may not be provided with an insertion hole. Instead, the user can utilize an awl, a drill or any other suitable tool to form an insertion hole 4 for the insertion of the stick in the stick insertion portion when it is to be used. In addition, it is also possible to provide, for example, a score, a material thinning portion, etc., in a suitable position on the stick insertion portion, so as to make it convenient for the user to form an insertion hole 4 there.

In order to prevent volatilization and/or leakage of the scented liquid in the diffuser bottle during transportation and storage, a volatilization-proof gasket 6 is provided on the upper cover portion of the safety inner cover 3. In an example, the volatilization-proof gasket 6 can be sealingly fixed on the upper cover portion 301 of the safety inner cover 3, and needs to be torn off before use. In another example, when the outer cover 2 is applied on the bottle body 1, the outer cover 2 can force the volatilization-proof gasket 6 to tightly press against the safety inner cover 3.

With the reed diffuser container according to the first embodiment of the present disclosure before use, the outer cover 2 and the safety inner cover 3 are both tightly connected to the bottle body 1 such that the scented liquid would not flow out even if the bottle body 1 was turned over; and the safety inner cover 3 is exposed when in use. Since the protrusion of the safety inner cover 3 engages with the groove 5, it takes a large force of finger to separate the two. Therefore, such a structure can effectively prevent children from accidentally opening the safety inner cover 3. In addition, since the insertion hole 4 is substantially closed before the stick is inserted, and the insertion hole 4 is tightly connected with the stick after the stick is inserted, the scented liquid would not leak out at any time even if the bottle body 1 was accidentally overturned.

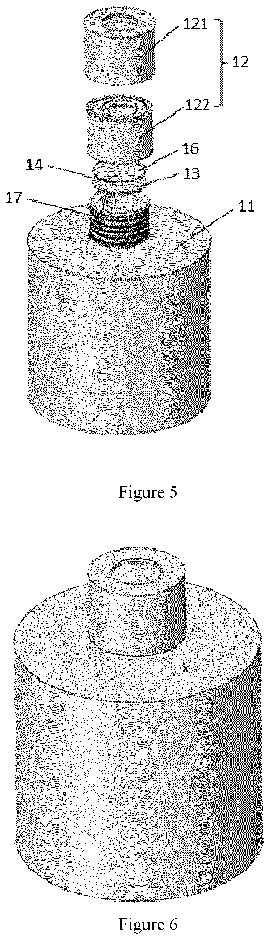

Figs. 5 to 8 illustrate a reed diffuser container according to a second embodiment of the present disclosure The reed diffuser container according to the second embodiment of the present disclosure includes a bottle body 11 for containing the scented liquid, wherein the bottle body 11 is provided with a neck, and the end of the neck is provided with a bottle mouth. External threads 17 are provided on the outer surface of the neck.

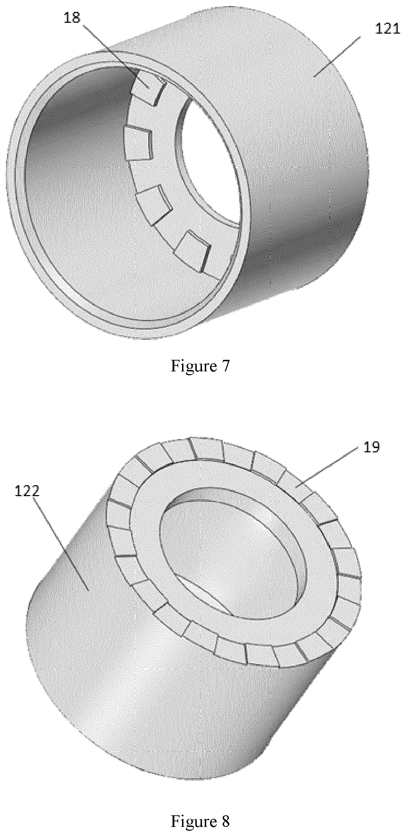

As shown in Fig. 5 and Fig. 6, an integral spinning-and-pressing type outer cover assembly 12 can be detachably connected with the bottle mouth of the bottle body 11. The outer cover assembly 12 includes a first cover member 121 (see Fig. 7) and a second cover member 122 (see Fig. 8). In use, the first cover member 121 is sleeved on the second cover member 122. Both the first cover member 121 and the second cover member 122 have an upper cover portion and a circumferentially extending skirt, and the respective upper cover portions of the first cover member 121 and the second cover member 122 are provided with aligned central through-holes. The inner surface of the skirt of the second cover member 122 is provided with internal threads matching the external threads 17 on the neck of the bottle body 11, such that the second cover member 122 can be thread-connected with the bottle body 11.

With further reference to Figs. 7 and 8, the inner surface of the upper cover portion of the first cover member 121 is provided with one or more protruding snap-fit portions 18, and the outer surface of the upper cover portion of the second cover member 122 is provided with one or more corresponding recessed portions 19. The snap-fit portion 18 can match with the recessed portion 19. The first cover member 121 is configured to be rotatable relative to the second cover member 122 and movable in the longitudinal direction when not engaged with the second cover member 122. When the first cover member 121 is rotated relative to the second cover member 122 such that the position of the snap-fit portions 18 corresponds to that of the recessed portions 19, the first cover member 121 can be moved downward in the longitudinal direction, whereby the snap-fit portion 18 is embedded into the recessed portion 19, and the first cover member 121 is snap-fitted with the second cover member 122, entering an engaged state. Further rotating the first cover member 121 when the first cover member 121 and the second cover member 122 are kept engaged enables the first cover member 121 to bring the second cover member 122 to rotate together, so that the outer cover assembly 12 can engage or disengage with the bottle body 11 through the thread connection between the internal threads on the second cover member 122 and the external threads 17 on the bottle body 11.

It will be understood by those skilled in the art that although not shown, the protruding snap-fit portion 18 may also be provided on the outer surface of the upper cover portion of the second cover member 122, while the recessed portion 19 may be provided on the inner surface of the upper cover portion of the first cover member 121 correspondingly. Other snap-fit features may also be used. In addition, various snap-fit features may also be provided on the surfaces of the skirts of the first cover member 121 and the second cover member 122 as needed.

In addition, in an example of the present disclosure the first cover member 121 and the second cover member 122 may be positioned at a distance from each other, and the first cover member 121 may be moved toward the second cover member 122 under the action of a longitudinal external force. This can be achieved, for example, by means of an elastic member arranged there between.

The bottle mouth of the bottle body 11 is provided with a safety inner cover 13, which may be made of an elastomer material such as silicone, rubber, etc. and tightly closes the bottle mouth of the bottle body 11 in a liquid-tight manner. In the illustrated embodiment, the safety inner cover 13 is in the form of a U-shaped plug with its circumferential flange overlying the rim of the bottle mouth.

Similar to the safety inner cover 3 of the first embodiment, the safety inner cover 13 is also provided with a stick insertion portion through which the sticks as carriers can be inserted into the bottle body 11 and in contact with the scented liquid in the bottle body 11. The position of the stick insertion portion corresponds to the central through-holes on the upper cover portions of the first cover member 121 and the second cover member 122. In the illustrated embodiment, the stick insertion portion includes a plurality of insertion holes 14. The configurations of the stick insertion portion and the insertion holes 14 are substantially the same as those of the stick insertion portion and the insertion holes 4 in the first embodiment, and the same features will not be repeated here.

Preferably, the diameter of the insertion holes 14 is 0.5 mm-5 mm, and the number is 2-20. More preferably, the diameter of the insertion holes 14 is less than or equal to 1.5 mm, and the number is 3-8. In the most preferred embodiment, the diameter of the insertion holes 14 is less than 1.5 mm.

In order to prevent volatilization and/or leakage of the scented liquid in the diffuser bottle through the insertion holes 14 during transportation and storage, a volatilization-proof gasket 16 is provided above the safety inner cover 3. When the second cover member 122 of the outer cover assembly 12 closes the bottle mouth, the inner surface of the upper cover portion of the second cover member 122 presses the volatilization-proof gasket 16 against the safety inner cover 13.

With the reed diffuser container according to the second embodiment of the present disclosure before use, the snap-fit portion 18 is separated from the recessed portion 19, and the outer cover assembly 12 cannot be separated from the bottle body 11. The outer cover assembly 12 can be rotated and removed as a whole only when a force is applied to keep the snap-fit portion 18 and the recessed portion 19 in an engaged state. Since such an opening step requires fine hand movements, it can effectively prevent children from accidentally opening the outer cover assembly 12 and causing the scented liquid to leak out. In addition, when the outer cover assembly 12 is removed, removing the volatilization-proof gasket 16 can have the safety inner cover 13 exposed. Then the outer cover assembly 12 can be used to closes the bottle body 11 again, and the sticks can be inserted through the through-holes on the first cover member 121 and the second cover member 122 and the insertion holes 14 of the reed insertion portion, so as to initiate the use of the product. Since the insertion holes 14 fit tightly with the reeds, the scented liquid would not leak out even if the bottle body 11 was accidentally overturned when in use.

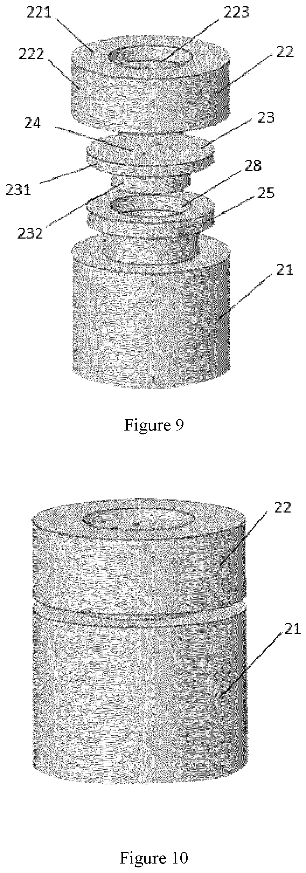

Figs. 9 to 10 illustrate a reed diffuser container according to a third embodiment of the present disclosure The reed diffuser container according to the third embodiment of the present invention comprises a bottle body 21 for containing the scented liquid, an outer cover 22 and a safety inner cover 23, wherein the bottle body 21 is provided with a neck; the end of the neck is provided with a bottle mouth 28; and the bottle mouth 28 is provided with a circumferential flange 25.

The safety inner cover 23 may be made of an elastomeric material such as rubber, and generally comprises an upper cover portion 231 and a plunger portion 232, wherein the plunger portion 232 can extend into the bottle mouth 28 of the bottle body 21 and fit closely with the inner wall of the bottle mouth 28 in a liquid-tight manner. The upper cover portion 231 is in the form of a platform positioned above the plunger portion 232 and has a diameter larger than that of the plunger portion 232. In an assembled state, the lower surface of the upper cover portion 231 tightly bears against the upper surface of the flange 25. According to the structure of the present invention, the upper cover portion 231 can better fix the safety inner cover 23 to the bottle mouth 28 of the bottle body 21, and the plunger portion 232 can be used to seal the bottle mouth 28.

The outer cover 22 comprises an upper cover portion 221 and a circumferentially extending skirt 222. The upper cover portion 221 of the outer cover 22 is provided with a central through-hole 223. The inner surface of the skirt 222 of the outer cover 22 is provided with a protrusion, which can be engaged beneath the flange 25 of the bottle body 21 such that the outer cover 22 covers the bottle mouth 28 of the bottle body 21. In a closed state, the upper cover portion 221 of the outer cover 22 tightly bear against the upper surface of the upper cover portion 231 of the safety inner cover 23, and the safety inner cover 23 is exposed through the through-hole 223; the skirt 222 covers all sides of the safety inner cover 23 and the flange 25 of the bottle body 21 to further seal the gap between the safety inner cover 23 and the bottle body 21.

Preferably, the outer cover is an integrally formed monolithic element. Preferably, the outer cover 22 is made of a metal sheet such as aluminum foil.

The safety inner cover 23 is provided with a reed insertion portion at a position corresponding to the through-hole 223, so that the sticks as carriers can be inserted into the bottle body 21 through the stick insertion portion, and in contact with the scented liquid in the bottle body 21. In the illustrated embodiment, the stick insertion portion includes a plurality of insertion holes 24. The configurations of the stick insertion portion and the insertion holes 24 are substantially the same as those of the stick insertion portion and the insertion holes 4 in the first embodiment, and the same features will not be repeated here.

Preferably, the insertion holes 24 are configured as blind holes and can be pierced prior to use, thereby allowing the stick to be inserted through the safety inner cover 23 into the bottle body 21.

Preferably, the plunger portion 232 is a hollow structure, and the blind holes are provided on the upper cover portion 231 at a position corresponding to the hollow portion of the plunger portion 232 to facilitate the piercing of the blind holes.

Preferably, in the case where the insertion holes 24 are configured as through-holes, a pierceable sealing sheet covering the stick insertion portion is additionally provided to avoid leakage of the scented liquid when the product is not in use.

Preferably, the safety inner cover 23 is an integrally formed monolithic element.

Preferably, means that can be used to pierce blind holes and/or sealing sheets such as an awl, a needle or the like can be connected to the bottle body 21 to facilitate the use of the reed diffuser container.

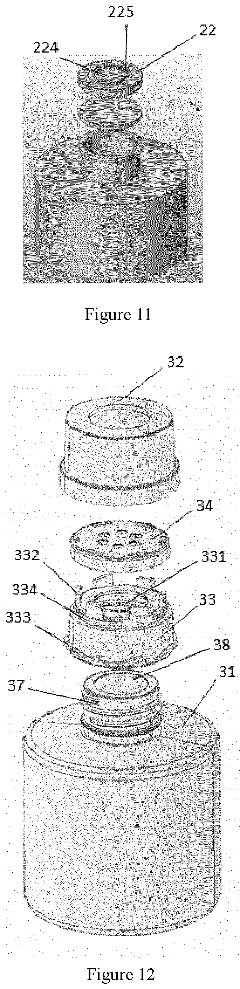

Referring to Fig. 11, in another example, the central through-hole 223 in the upper cover portion 221 of the outer cover 22 can be initially sealed by a cover sheet 224. The cover sheet 224 is connected to the periphery of the central through-hole 223 through a frangible connection, and a pull ring 225 is provided on the upper surface thereof. When in use, the user can remove the cover sheet 224 by pulling the pull ring 225 to expose the central through-hole 223 and thereby expose the safety inner cover 23.

With the reed diffuser container of the third embodiment of the present disclosure since the outer cover 22 is tightly wrapped around the safety inner cover 23 and the bottle mouth flange 25, children will not be able to remove the outer cover 22 from the bottle body 21 and cause danger. When in use, the user only needs to conveniently use the attached awl or any other suitable means to penetrate the insertion holes 24, and the sticks can then be inserted into the bottle body 21 to initiate the use of the product. Since the insertion holes 24 are designed to abut closely with the stick, the scented liquid would not leak out even if the bottle body 11 was accidentally overturned when in use.

Figs. 12 to 15 illustrate a reed diffuser container according to a fourth embodiment of the present disclosure The reed diffuser container according to the fourth embodiment of the present disclosure includes a bottle body 31 for containing the scented liquid, wherein the bottle body 31 is provided with a neck; the end of the neck is provided with a bottle mouth 38; external threads 37 are provided on the neck of the bottle body 31; the mouth 38 of the bottle body 31 is sealed by an assembly including an outer cover 32, an inner cover 33 and an intermediate sealing cover 34.

The upper cover portion of the inner cover 33 is provided with a central through-hole 331, and the inner surface of its circumferentially extending skirt is provided with internal threads that match the external threads 37 on the neck of the bottle body 31 such that the inner cover 33 can be thread-connected with the bottle body 31.

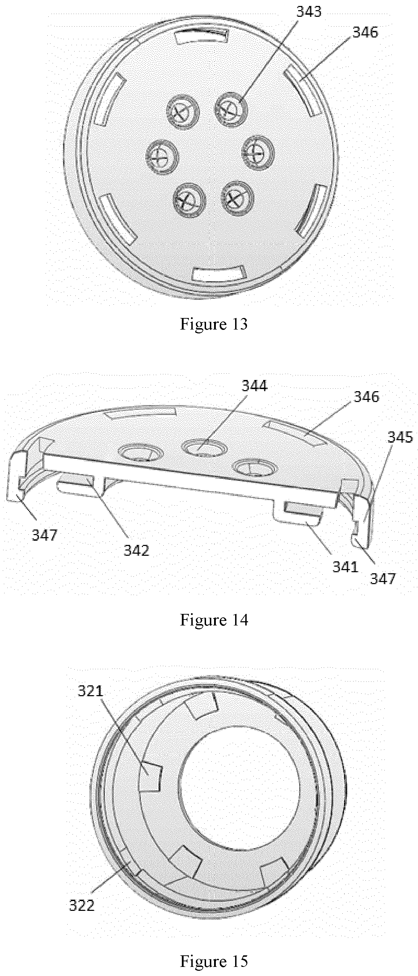

The intermediate sealing cover 34 may be positioned above the upper cover portion of the inner cover 33 in a liquid-tight manner and covers the central through-hole 331 thereof. Figs. 13 and 14 specifically show the detailed configuration of the intermediate sealing cover 34. The intermediate sealing cover 34 has a substantially disc-shaped body. The sealing portion 341 extends radially outward from the lower surface of the body, thereby forming an engaging groove 342 with the lower surface of the body. The intermediate sealing cover 34 may be made of an elastomeric material such as silicone, rubber, or the like. During assembly, the intermediate sealing cover 34 is pushed toward the inner cover 33 by force such that the sealing portion 341 extends into the inner cover 33 through the through-hole 331 on the inner cover 33, and the engaging groove 342 is snap-fitted with the edge of the through-hole 331, whereby the intermediate sealing cover 34 is tightly mounted on the upper cover portion of the inner cover 33. The edge of the intermediate sealing cover 34 is circumferentially provided with mounting slots 346 that allow the engaging tabs 332 on the inner cover 33, which are described in more detail below, to pass there through, thereby limiting the relative rotation between the inner cover 33 and the intermediate sealing cover 34. In addition, the intermediate sealing cover 34 is further provided with a wrapping portion 345 extending downward from the edge of the disc-shaped body. The lower end flange 347 of the wrapping portion 345 can be positioned in the groove 334 in the upper portion of the inner cover 33 in a form-fitting manner to further fix the intermediate sealing cover 34 to the inner cover 33. Since the wrapping portion 345 wraps the outer edge of the upper cover portion of the inner cover 33, better sealing can be obtained effectively.

The intermediate sealing cover 34 further includes a stick insertion portion. The upper cover portion of the outer cover 32 is provided with a central through-hole aligned with the central through-hole 331, and during assembly, the stick insertion portion can be exposed through the central through-hole. The stick insertion portion includes a plurality of insertion holes 344. The configurations of the stick insertion portion and the insertion holes 344 may be substantially the same as those of the stick insertion portion and the insertion holes 4 in the first embodiment, and the same features will not be repeated here.

Preferably, in the case where the insertion holes 344 are configured as through-holes, a pierceable sealing sheet covering the stick insertion portion is additionally provided to avoid leakage of the scented liquid.

In the example shown in Figs. 13 and 14, the insertion holes 344 are configured as pierceable blind holes. The figures also show cross scores provided at the bottom of the blind holes, which can facilitate easy penetration of the blind holes by the sticks.

Engaging tabs 332 are provided in the upper cover portion of the inner cover 33, and engaging protrusions 333 are provided on the outer surface of the circumferentially extending skirt at the lower end of the inner cover 33. Correspondingly, as shown in Fig. 15, bosses 321 are provided on the inner surface of the upper cover portion of the outer cover 32, and a bottom portion of the inner surface of the circumferentially extending skirt of the outer cover 32 is provided with engaging receptacles 322 which match with the engaging protrusions 333 on the inner cover 33.

During assembly, the inner cover 33 is sleeved inside the outer cover 32, and the outer cover 32 is rotatable relative to the inner cover 33 and movable in the longitudinal direction. The inner cover 33 and the outer cover 32 are configured to be at a distance from each other in the longitudinal direction when not in use. At this time, the outer cover 32 moves independently of the inner cover 33. When the outer cover 32 is pressed down by force, the outer cover 32 comes close to the inner cover 33. When the outer cover 32 is rotated relative to the inner cover 33 such that the engaging tabs 332 of the inner cover 33 abut against the bosses 321 of the outer cover 32 and that the engaging protrusions 333 of the inner cover 33 are engaged with the engaging receptacles 322 of the outer cover 32, the inner cover 33 can be rotated together with the outer cover 32 such that the inner cover 33 can be threaded-connected or disconnected with the bottle mouth 38.

It will be understood by those skilled in the art that although not shown in the present disclosure the engagement between the inner cover 33 and the outer cover 32 can be achieved by various other engaging features. In addition, the engagement between the engaging tabs 332 of the inner cover 33 and the bosses 321 of the outer cover 32 and the engagement between the engaging protrusions 333 of the inner cover 33 and the engaging receptacles 322 of the outer cover 32 are not necessary to present at the same time, i.e., one of them will suffice.

With the reed diffuser container according to the fourth embodiment of the present disclosure before use, the inner cover 33 is separated from the outer cover 32, and the inner cover 33 cannot be separated from the bottle body 31. The inner cover 33 and the outer cover 32 can be rotated as a whole only when a force is applied to keep the engaging tabs 332 and the engaging protrusions 333 of the inner cover 33 and the bosses 321 and the engaging receptacles 322 of the outer cover 32 in an engaged state. Since such an opening step requires fine hand movements, it can effectively prevent children from accidentally opening the inner cover 33 and cause the scented liquid to leak out. In addition, when in use, the user only needs to use the attached awl or any other suitable means to penetrate the insertion holes 344, and then insert the stick into the bottle body 31 to initiate the use of the product. Since the insertion holes 344 are designed to abut closely with the sticks, the scented liquid would not leak out even if the bottle body 31 was accidentally overturned in use.

It will be understood by those skilled in the art that while this disclosure discusses specific embodiments with respect to scented liquid, the disclosed containers and their closures can be used for a variety of substances, especially evaporative volatile substances including fragrances and/or actives, that are conveyed and dispensed through a wicking action. In addition to liquid substances, the present invention can also be used in particular for gelatinous substances or other suitable substances to be dispensed.

It will also be understood by those skilled in the art that although this disclosure discusses specific embodiments with respect to the stick, it is also possible to use cotton strings, wicking tubes, fiber sticks, rattan and any other wicking members that can utilize capillary action to convey the substance to be dispensed.

It will be understood by those skilled in the art that only structures deemed necessary to illustrate the container are described herein. Furthermore, while several examples of the reed diffuser container have been described above, all features contained in the above description or shown in the drawings are for illustrative purposes only and are not intended to limit the present disclosure. The skilled person may implement various design variations without departing from the scope of the invention as defined in the claims.

## Claims

1. A reed diffuser container, which includes a bottle body (11) for containing the scented liquid, wherein the bottle body (11) is provided with a bottle mouth (8); the container further comprises a safety inner cover (13), which is provided with a stick insertion portion to allow sticks to pass through the safety inner cover (13) and enter the bottle body (11); the stick insertion portion includes one or more insertion holes (14), or one or more insertion holes (14) can be formed on the stick insertion portion; the insertion hole (14) is configured such that at least a part of its wall abuts closely with an inserted stick; the insertion holes (14) can be either through-holes or blind holes,
**characterized in that** the safety inner cover (13) is in the form of a plug, and tightly closes the bottle mouth in a liquid-tight manner,
wherein an outer cover assembly (12) can be detachably connected to the bottle mouth (8) from the outside of the inner safety cover (13); the outer cover assembly (12) comprises a first cover member (121) and a second cover member (122); when in use, the first cover member (121) is sleeved outside of the second cover member (122); both the first cover member (121) and the second cover member (122) have an upper cover portion and a circumferentially extending skirt, and the upper cover portions of the first cover member (121) and the second cover member (122) are provided with aligned central through-holes to expose the reed insertion portion;
wherein the first cover member (121) and the second cover member (122) are provided with matching engaging features (18, 19), through which the first cover member (121) and the second cover member (122) can engage with each other, such that in an engaged state, the second cover member (122) can rotate along with the rotation of the first cover member (121);
wherein in a disengaged state, the first cover member (121) is configured to be rotatable relative to the second cover member (122) and movable in the longitudinal direction,
wherein the engaging features (18, 19) comprise one or more protruding snap-fit portions (18) or recessed portions (19) on the inner surface of the upper cover portion of the first cover member, and one or more corresponding recessed portions (19) or protruding snap-fit (18) portions provided on the outer surface of the upper cover portion of the second cover member (122), and
wherein the engagement between the first cover member (121) and the second cover member (122) is achieved by pressing the first cover member (121) against the second cover member (122) and rotating the second cover member (122) such that the one or more protruding snap-fit portions (18) or recessed portions (19) of the first cover member (121) are embedded into the one or more recessed portions (19) or protruding snap-fit portions (18) of the second cover member (122).

2. The reed diffuser container according to claim 1, **characterized in that** a volatilization-proof gasket (16) is provided between the safety inner cover (13) and the second cover member (122).

3. The reed diffuser container according to claim 1, **characterized in that** the second cover member (122) can be threadedly connected with the bottle body (11).

4. The reed diffuser container according to any one of claims 1 to 3, **characterized in that** the inner safety cover (13) is made of an elastomeric material.

5. The reed diffuser container according to claim 1, **characterized in that** the insertion holes are configured as through-holes and are in a closed state before the sticks are inserted.

6. The reed diffuser container according to claim 5, **characterized in that** the insertion hole comprises at least one of the following features:
1) the diameter of the insertion hole is smaller than the diameter of the stick;
2) the extension direction of the insertion hole is inclined with respect to the longitudinal direction of the bottle body.

## Patentansprüche

1. Duftstäbchenhalter, der einen Flaschenkörper (11) zum Aufnehmen der Duftflüssigkeit umfasst, wobei der Flaschenkörper (11) mit einer Flaschenmündung (8) versehen ist; wobei der Halter ferner eine innere Sicherheitsabdeckung (13) umfasst, die mit einem Stäbcheneinsetzabschnitt versehen ist, damit Stäbchen die innere Sicherheitsabdeckung (13) passieren und in den Flaschenkörper (11) eintreten können; wobei der Stäbcheneinsetzabschnitt ein oder mehrere Einsetzlöcher (14) umfasst oder ein oder mehrere Einsetzlöcher (14) am Stäbcheneinsetzabschnitt ausgebildet werden können; wobei das Einsetzloch (14) so ausgelegt ist, dass zumindest ein Teil seiner Wand eng an einem eingesetzten Stäbchen anliegt; wobei die Einsetzlöcher (14) entweder Durchgangslöcher oder Sacklöcher sein können,
**dadurch gekennzeichnet, dass** die innere Sicherheitsabdeckung (13) als Stopfen ausgebildet ist und die Flaschenmündung flüssigkeitsdicht verschließt, wobei eine äußere Abdeckungsanordnung (12) von der Außenseite der inneren Sicherheitsabdeckung (13) lösbar mit der Flaschenmündung (8) verbunden werden kann; wobei die äußere Abdeckungsanordnung (12) ein erstes Abdeckungselement (121) und ein zweites Abdeckungselement (122) umfasst; wobei bei der Verwendung das erste Abdeckungselement (121) außerhalb des zweiten Abdeckungselements (122) aufgesteckt ist; wobei sowohl das erste Abdeckungselement (121) als auch das zweite Abdeckungselement (122) einen oberen Abdeckungsabschnitt und eine sich in Umfangsrichtung erstreckende Schürze aufweisen und die oberen Abdeckungsabschnitte des ersten Abdeckungselements (121) und des zweiten Abdeckungselements (122) mit ausgerichteten mittigen Durchgangslöchern versehen sind, um den Stäbcheneinsetzabschnitt freizulegen;
wobei das erste Abdeckungselement (121) und das zweite Abdeckungselement (122) mit passenden Eingriffsmerkmalen (18, 19) versehen sind, durch die das erste Abdeckungselement (121) und das zweite Abdeckungselement (122) miteinander in Eingriff treten können, sodass sich das zweite Abdeckungselement (122) in einem Eingriffszustand zusammen mit der Drehung des ersten Abdeckungselements (121) drehen kann;
wobei in einem Lösezustand das erste Abdeckungselement (121) dazu ausgelegt ist, relativ zum zweiten Abdeckungselement (122) drehbar und in Längsrichtung beweglich zu sein,
wobei die Eingriffsmerkmale (18, 19) einen oder mehrere vorstehende Einrastabschnitte (18) oder vertiefte Abschnitte (19) auf der Innenfläche des oberen Abdeckungsabschnitts des ersten Abdeckungselements und einen oder mehrere entsprechende vertiefte Abschnitte (19) oder vorstehende Einrastabschnitte (18), die auf der Außenfläche des oberen Abdeckungsabschnitts des zweiten Abdeckungselements (122) vorgesehen sind, umfassen und wobei der Eingriff zwischen dem ersten Abdeckungselement (121) und dem zweiten Abdeckungselement (122) durch Drücken des ersten Abdeckungselements (121) gegen das zweite Abdeckungselement (122) und Drehen des zweiten Abdeckungselements (122), sodass der eine oder die mehreren vorstehenden Einrastabschnitte (18) oder vertieften Abschnitte (19) des ersten Abdeckungselements (121) in den einen oder die mehreren vertieften Abschnitte (19) oder die vorstehenden Einrastabschnitte (18) des zweiten Abdeckungselements (122) eingebettet sind, erreicht wird.

2. Duftstäbchenhalter nach Anspruch 1, **dadurch gekennzeichnet, dass** zwischen der inneren Sicherheitsabdeckung (13) und dem zweiten Abdeckungselement (122) eine verdunstungssichere Dichtung (16) vorgesehen ist.

3. Duftstäbchenhalter nach Anspruch 1, **dadurch gekennzeichnet, dass** das zweite Abdeckungselement (122) mit dem Flaschenkörper (11) verschraubbar ist.

4. Duftstäbchenhalter nach einem der Ansprüche 1 bis 3, **dadurch gekennzeichnet, dass** die innere Sicherheitsabdeckung (13) aus einem elastomeren Material besteht.

5. Duftstäbchenhalter nach Anspruch 1, **dadurch gekennzeichnet, dass** die Einsetzlöcher als Durchgangslöcher ausgebildet sind und sich vor dem Einsetzen der Stäbchen in einem geschlossenen Zustand befinden.

6. Duftstäbchenhalter nach Anspruch 5, **dadurch gekennzeichnet, dass** das Einsetzloch mindestens eines der folgenden Merkmale umfasst:
1) der Durchmesser des Einsetzlochs ist kleiner als der Durchmesser des Stäbchens;
2) die Ausdehnungsrichtung des Einsetzlochs ist bezüglich der Längsrichtung des Flaschenkörpers geneigt.

## Revendications

1. Récipient diffuseur à roseaux, qui comprend un corps de flacon (11) destiné à contenir le liquide parfumé, le corps de flacon (11) étant pourvu d'un goulot de flacon (8) ; le récipient comprenant en outre un couvercle interne de sécurité (13) qui est pourvu d'une partie d'insertion de bâtonnet pour permettre à des bâtonnets de passer à travers le couvercle interne de sécurité (13) et d'entrer dans le corps de flacon (11) ; la partie d'insertion de bâtonnet comprenant un ou plusieurs trous d'insertion (14), ou un ou plusieurs trous d'insertion (14) pouvant être formés sur la partie d'insertion de bâtonnet ; le trou d'insertion (14) étant conçu de sorte qu'au moins une partie de sa paroi vienne étroitement en butée avec un bâtonnet inséré ; les trous d'insertion (14) pouvant être des trous traversants ou des trous borgnes,
**caractérisé en ce que** le couvercle interne de sécurité (13) a la forme d'un bouchon et ferme de manière étanche le goulot de flacon,
un ensemble couvercle externe (12) pouvant être relié de manière amovible au goulot de flacon (8) depuis l'extérieur du couvercle de sécurité interne (13) ; l'ensemble couvercle externe (12) comprenant un premier élément de couvercle (121) et un second élément de couvercle (122) ; lors de l'utilisation, le premier élément de couvercle (121) étant emmanché à l'extérieur du second élément de couvercle (122) ; le premier élément de couvercle (121) et le second élément de couvercle (122) ayant tous deux une partie de couvercle supérieure et une jupe s'étendant circonférentiellement, et les parties de couvercle supérieures du premier élément de couvercle (121) et du second élément de couvercle (122) étant pourvues de trous traversants centraux alignés pour exposer la partie d'insertion de roseau ;
le premier élément de couvercle (121) et le second élément de couvercle (122) étant pourvus de caractéristiques de mise en prise correspondantes (18, 19), à travers lesquelles le premier élément de couvercle (121) et le second élément de couvercle (122) peuvent venir en prise l'un avec l'autre, de sorte que, dans un état en prise, le second élément de couvercle (122) puisse tourner avec la rotation du premier élément de couvercle (121) ;
dans un état séparé, le premier élément de couvercle (121) étant conçu pour pouvoir tourner par rapport au second élément de couvercle (122) et étant mobile dans la direction longitudinale,
les caractéristiques de mise en prise (18, 19) comprenant une ou plusieurs parties d'encliquetage saillantes (18) ou parties enfoncées (19) sur la surface interne de la partie de couvercle supérieure du premier élément de couvercle, et une ou plusieurs parties enfoncées (19) ou parties d'encliquetage saillantes (18) correspondantes situées sur la surface externe de la partie de couvercle supérieure du second élément de couvercle (122), et
la mise en prise entre le premier élément de couvercle (121) et le second élément de couvercle (122) étant obtenue en pressant le premier élément de couvercle (121) contre le second élément de couvercle (122) et en faisant tourner le second élément de couvercle (122) de sorte que la ou les parties à encliquetage saillantes (18) ou les parties enfoncées (19) du premier élément de couvercle (121) soient intégrées dans la ou les parties enfoncées (19) ou parties à encliquetage saillantes (18) du second élément de couvercle (122).

2. Récipient diffuseur à roseaux selon la revendication 1, **caractérisé en ce qu'**un joint étanche à la volatilisation (16) est fourni entre le couvercle interne de sécurité (13) et le second élément de couvercle (122).

3. Récipient diffuseur à roseaux selon la revendication 1, **caractérisé en ce que** le second élément de couvercle (122) peut être relié par vissage au corps de flacon (11).

4. Récipient diffuseur à roseaux selon l'une quelconque des revendications 1 à 3, **caractérisé en ce que** le couvercle de sécurité interne (13) est en un matériau élastomère.

5. Récipient diffuseur à roseaux selon la revendication 1, **caractérisé en ce que** les trous d'insertion sont réalisés sous la forme de trous traversants et se trouvent dans un état fermé avant l'insertion des bâtonnets.

6. Récipient diffuseur à roseaux selon la revendication 5, **caractérisé en ce que** le trou d'insertion comprend au moins l'une des caractéristiques suivantes :
1) le diamètre du trou d'insertion est inférieur au diamètre du bâtonnet ;
2) la direction d'extension du trou d'insertion est inclinée par rapport à la direction longitudinale du corps de flacon.
